# EUROPEAN PATENT APPLICATION

(11) **EP 1 932 563 A1**
(43) Date of publication of application: **18.06.2008**
(21) Application number: 07023316.8
(22) Date of filing: 01.12.2007
(51) Int. Cl.: A61N 1/37

(54) **Implantable medical device**

(30) Priority: 15.12.2006 US 611449
(71) Applicant: BIOTRONIK CRM Patent AG, 6341 Baar (CH)
(72) Inventor: Garner, Garth, Tigart, OR 97224 (US); Kreidler, Christian, Portland, OR 97202 (US); Nigam, Indra, B., 97223 Tigart, OR 97223 (US)
(74) Representative: Lindner-Vogt, Karin L.

(57) **Abstract**

The invention relates to an implantable medical device for processing picked-up electric heart signals and to a method of controlling sensitivity of a sensing stage of the device.

The sensing stage is connected to an electrode picking up electric potentials inside the heart, has an adjustable sensitivity and generates a detect signal indicating detection of a sense event if the time course of the picked-up signal exceeds the detection threshold.

A peak amplitude detector determines a peak amplitude, a peak amplitude comparator determines whether the peak amplitude exceeds a predetermined peak amplitude threshold value, and a control unit adapts the detection threshold by adjusting the sensing stage's sensitivity. The control unit sets the detection threshold for a first detection period to a high value, subsequently lowers the detection threshold stepwise for further detection periods.

## Description

The invention refers to an implantable medical device (IMD) providing means for sensing intracardiac electric potentials and an automatic sensitivity control for said sensing means. Typically such implantable medical device is a heart stimulator such as an implantable cardiac pacemakers and/or an implantable cardioverter/defibrillator (ICD).

Implantable heart stimulators can be used for treating a variety of heart disorders like bradycardia, tachycardia or fibrillation.

Depending on the disorder to be treated, such heart stimulator generates electrical stimulation pulses that are delivered to the heart tissue (myocardium) of a respective heart chamber according to an adequate timing regime. Delivery of stimulation pulses to the myocardium is usually achieved by means of an electrode lead that is electrically connected to a stimulation pulse generator inside a heart stimulator's housing and that carries a stimulation electrode in the region of it's distal end. A stimulation pulse having strong enough a strength causes an excitation of the myocardium that in turn is followed by a contraction of the respective heart chamber. A stimulation pulse also is called a pace. Similarly, pacing a heart chamber means stimulating a heart chamber by delivery of a stimulation pulse.

In order to be able to sense a contraction of a heart chamber that naturally occurs without artificial stimulation (also called intrinsic contraction), the heart stimulator usually comprises at least one sensing stage that is connected to a sensing electrode on said electrode placed in the heart chamber. An intrinsic excitation of a heart chamber results in characteristic electrical potentials that can be picked up via the sensing electrode and that can be evaluated by the sensing stage in order to determine whether an intrinsic excitation - called: intrinsic event - has occurred. In order to detect an intrinsic excitation of a heart chamber the picked-up signal (referred to as "heart signal" henceforth) is amplified by an amplifier of the sensing stage. The amplifiers gain usually is adjustable. The sensing stage usually further comprises a comparator for comparing the amplified heart signal with a reference value. If the amplified signal exceeds the reference value a detect signal indicating detection of an (intrinsic) event is generated. The sensitivity of the sensing stage hence depends on both, the amplifier's gain and the comparator's threshold as given by the reference value. The detection threshold of the sensing stage thus is determined by the sensing stage's sensitivity. In order to detect all intrinsic excitation while suppressing e.g. noise even if the picked-up signal is fading the detection threshold needs to be adapted by adjusting the sensitivity of the sensing stage accordingly. Adjustment of the sensing stage's sensitivity can be achieved by adjusting the amplifier's gain or by adjusting the comparator's threshold or both. Accordingly, adaptation or adjustment of the sensing stage's sensitivity determining the detection threshold shall refer to any possible technical implementation.

In an implantable pacemaker or defibrillator, the heart signal is sensed using unipolar or bipolar electrode leads where at least the tip electrode is in contact with the heart tissue in order to pick up the heart signal.

Typically, the heart signal is sensed in different chambers of the heart - left and/or right atrium and/or ventricle and hence a plurality of sensing channels having individual sensing stages are provided. In a dual chamber pacemaker usually two separate sensing stages, an atrial sensing stage and a ventricular sensing stage , are provided that are capable to detect intrinsic atrial events Aₛ (atrial sensed event) or intrinsic ventricular events Vₛ (ventricular sensed event), respectively. The signal picked-up, e.g. from the right ventricle, includes the QRS complex as well as T- and U-waves. The object of an Automatic Sensitivity Control (ASC) feature for the right ventricle is to sense each QRS complex and avoid sensing T- and U-waves. Similarly, it is desirable to sense only the P-waves in the right atrium. In the case of a tachyarrhythmia-detecting device, it is important to be able to detect low-level fibrillation signals while avoiding unwanted portions of the signal and noise.

The above mentioned object can be achieved by a combination of appropriate signal filtering targeted at attenuating the noise and other unwanted signal portions and dynamic adjustment of the sensing threshold which is adapted to the amplitude of the detected heart complex. A prior art embodiment of an implantable medical device providing automatic gain control is disclosed in US 5,891,048. US 5,891,048 is considered to constitute the closest prior for this application. Other prior art is given by US 4,940,054, US 5,117,824 and US 5, 339,820.

In addition to the sensing channels mentioned above an implantable pacemaker or defibrillator features separate stimulation generators for each heart chamber to be stimulated. Therefore, in a dual chamber pacemaker, usually an atrial and a ventricular stimulation pulse generator for generating atrial and ventricular stimulation pulses are provided. Delivery of an atrial or a ventricular stimulation pulse causing an artificial excitation of the atrium or the ventricle, respectively, is called an atrial stimulation event Aₚ (atrial paced event) or a ventricular stimulation event Vₚ (ventricular paced event), respectively.

By means of a sensing stage for a heart chamber to be stimulated, the pacemaker is able to only trigger stimulation pulses when needed that is when no intrinsic excitation of the heart chamber occurs in time. Such mode of pacing a heart chamber is called demand mode. In the demand mode the pacemaker schedules an atrial or a ventricular escape interval that causes triggering of an atrial or ventricular stimulation pulses when the escape interval times out. Otherwise, if an intrinsic atrial or ventricular event is detected prior to time out of the respective atrial or ventricular escape interval, triggering of the atrial or ventricular stimulation pulse is inhibited.

Depending upon which chambers of heart are stimulated and which sense events are used different modes of stimulation become available. These modes of stimulation are commonly identified by a three letter code wherein the first letter identifies the chamber or chambers to be stimulated such as V for a ventricle to be stimulated, A for an atrium to be stimulated and D (dual) for both, ventricle and atrium to be stimulated. Similarly, the second letter characterizes the chamber or chambers sensed events may origin from (V: ventricle, A: atrium, D: ventricle and atrium). The third letter characterizes the mode of delivery of stimulation pulses: T = triggered, I = inhibited and D = dual (T + I). A fourth letter "R" may characterize a rate adaptive heart stimulator that comprises an activity sensor or some other means for determining the hemodynamic need of a patient in or to adapt the stimulation rate accordingly.

It is an object of the invention to provide an implantable medical device that provides a means for automatic sensitivity control that provides for reliable detection of any event to be sensed such as a R-wave in the ventricle or a P-wave in the atrium while being unsusceptible to any false detection due to noise etc..

According to the present invention the object of the invention is achieved by an implantable medical device featuring:
a sensing stage connected or being connectable to an electrode for picking up electric potentials inside at least one chamber of a heart, said sensing stage having an adjustable sensitivity determining detection threshold, said sensing stage being further adapted to generate a detect signal indicating a sense event if the time course of the picked-up signal exceeds the detection threshold, and
a control that is connected to the sensing stage and that is adapted to adapt the detection threshold by adjusting the sensing stage's sensitivity as follows:
   set the detection threshold to a high value corresponding to low sensitivity of the sensing stage for a first detection period following a detect signal generated by the sensing stage, and
   subsequently lower the detection threshold stepwise for further detection periods
   wherein either a first duration is used for the first detection period following a heart signal having an amplitude below the preset peak amplitude threshold or
   a second duration is used for the first detection period following a heart signal having an amplitude above the preset peak amplitude threshold, said first duration being longer than said second duration.

Preferably, the implantable medical device further features a stimulation pulse generator adapted to generate electric stimulation pulses and being connected or being connectable to at least a ventricular stimulation electrode for delivering electric stimulation pulses to at least said ventricle of the heart, and the control unit preferably is connected to said stimulation pulse generator and is adapted to trigger the stimulation pulse generator to deliver stimulation pulses at scheduled points of time determined by the control unit.

Preferably, the high value of the detection threshold set after a detect signal is generated by the sensing stage is equal to a value that is a function of the peak amplitude of the picked up heart signal as determined by the peak amplitude detector.

In a preferred embodiment, the control unit is adapted to apply the first (longer) duration for the first detection period following the delivery of a stimulation pulse. The control unit preferably is further adapted to:
set a minimum detection threshold limit high enough to avoid detection of noise;
set a peak amplitude threshold for discrimination of large peak amplitude heart signals from low peak amplitude heart signals;
start a hold-off period prior to the first detection period each time the amplitude of a picked-up heart signal exceeds an actual detection threshold during which
   (i) no detection can be made,
   (ii) the peak amplitude of the heart signal is determined, and
   (iii) the detected peak amplitude is categorized as to whether or not the peak amplitude exceeds said peak amplitude threshold value by comparing the detected peak amplitude against the said peak amplitude threshold value;
following the expiration of the hold-off period, to adjust the detection threshold to be equal to a value that is a function of the peak amplitude of the complex as searched and found according to the above;
subsequently to lower the detection threshold for each detection period gradually to reach a lower final detection threshold value, the said lower detection threshold value being a function of the said detected peak amplitude value but never being lower than the said minimum detection threshold value;
start a hold-off period when a stimulation pulse is delivered during which no detection can be made;
adjust the detection threshold upon expiration of the hold-off period following a stimulation pulse to a fixed value high enough to avoid detecting evoked T-waves;
subsequently to lower the detection threshold gradually to reach the final threshold value which is set equal to the said minimum detection threshold limit; and
abort the lowering of the detection threshold value if the threshold before reaching said minimum detection threshold value when the heart signal exceeds the detection threshold or when a stimulation pulse is delivered.

Preferably, the implantable medical device further comprises a noise timer and a noise threshold comparator for comparing the heart signal with a noise threshold value, said noise timer having the effect that no further detections are possible as long as the noise timer is running, wherein the control unit is adapted to start noise timer at the same time when the control unit starts said hold-off period, and to restart said noise timer each time the polarity of the heart signal changes while the magnitude of it remains above a noise threshold, or the magnitude of the heart signal crosses the noise threshold from below.

Said noise timer preferably is adapted to keep its value frozen during any analog blanking that follows a delivery of a stimulation pulse or defibrillation shock.

The sensing stage preferably is adapted to sample the heart signal and to confirm detection of a sense event when more than one consecutive samples of the heart signal exceed the detection threshold.

The object of the invention also is achieved by a method of controlling sensitivity of a sensing stage of an implantable medical device as pointed out above.

The method comprising the steps of:
Setting a minimum detection threshold limit high enough to avoid detection of noise;
Setting a peak amplitude threshold for discrimination of large peak amplitude heart signals from low peak amplitude heart signals;
Starting a holdoff period each time the amplitude of a picked-up heart signal exceeds an actual detection threshold during which
   (i) no detection can be made,
   (ii) the peak amplitude of the heart signal is determined, and
   (iii) the detected peak amplitude is categorized as to whether or not the peak amplitude exceeds said peak amplitude threshold value by comparing the detected peak amplitude against the said peak amplitude threshold value;
      following the expiration of the hold-off period, Adjusting the detection threshold to be equal to a value that is a function of the peak amplitude of the complex as searched and found according to the above;
      subsequently Lowering the detection threshold gradually to reach a lower final detection threshold value, the said lower detection threshold value being a function of the said detected peak amplitude value but never being lower than the said minimum detection threshold value;
      Starting a hold-off period when a stimulation pulse is delivered during which no detection can be made;
      Adjusting the detection threshold upon expiration of the holdoff period following a stimulation pulse to a fixed value high enough to avoid detecting most evoked T-waves;
      subsequently lowering the detection threshold gradually to reach the final detection threshold value which is set equal to the said minimum detection threshold limit; and
      Aborting the lowering of the detection threshold value if the threshold before reaching said minimum detection threshold value when the heart signal exceeds the detection threshold or when a stimulation pulse is delivered.

The control unit of an implantable medical device is preferably adapted to perform said method.

The concept of providing an implantable medical device that is adapted to utilize the information related to the type or classification of the sensed or paced heart event to decide whether or not to adapt or adjust the final detection threshold value and In case of no adaptation or adjustment to use the previous value of the final detection threshold as the target detection threshold value when lowering the detection threshold can be used in combination with the feature previously discussed or independently from the othe features discussed herein.

According to a preferred embodiment, the final detection threshold value is not adapted or adjusted if one or more of the following event types are detected and instead a previous value of the final detection threshold value is used as a target detection threshold value when lowering the detection threshold:
- a single premature ventricular contraction or first premature ventricular contraction in a sequence of premature ventricular contractions,
- a single detection in the retrograde window or first detection in a sequence of such detections
- a Far-field detection of a ventricular complex in the atrium
- a delivery of a safety window pace pulse
- a delivery of a pace pulse while the said noise timer is running
- if noise is suspected at the expiry of the hold-off period following detection of a sense event.

Accordingly, a preferred embodiment of the implantable medical device comprises means for detecting at least one of the event types that shall prevent an adaptation of the final detection threshold value as listed above.

The above and other aspects, features and advantages of the present invention will be more apparent from the following more particular description thereof, presented in conjunction with the following drawings wherein:
- FIG. 1: shows a dual chamber pacemaker connected to leads placed in a heart.
- FIG. 2: is a block diagram of a heart stimulator according to the invention.

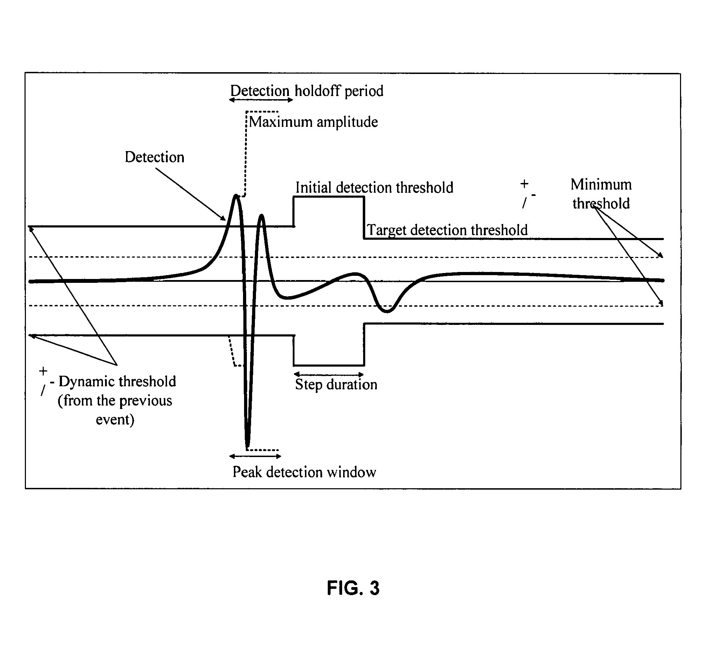

- FIG. 3: is an electrocardiogram representing a part of a ventricular heart signal including a QRS complex and a T-wave illustrating the setting of detection thresholds according to the invention.

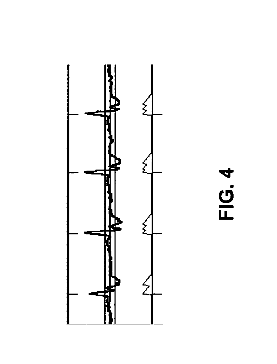

- FIG. 4: is a diagram illustrating retriggering of the noise filter according to a preferred embodiment.

The following description is of the best mode presently contemplated for carrying out the invention. This description is not to be taken in a limiting sense, but is made merely for the purpose of describing the general principles of the invention. The scope of the invention should be determined with reference to the claims.

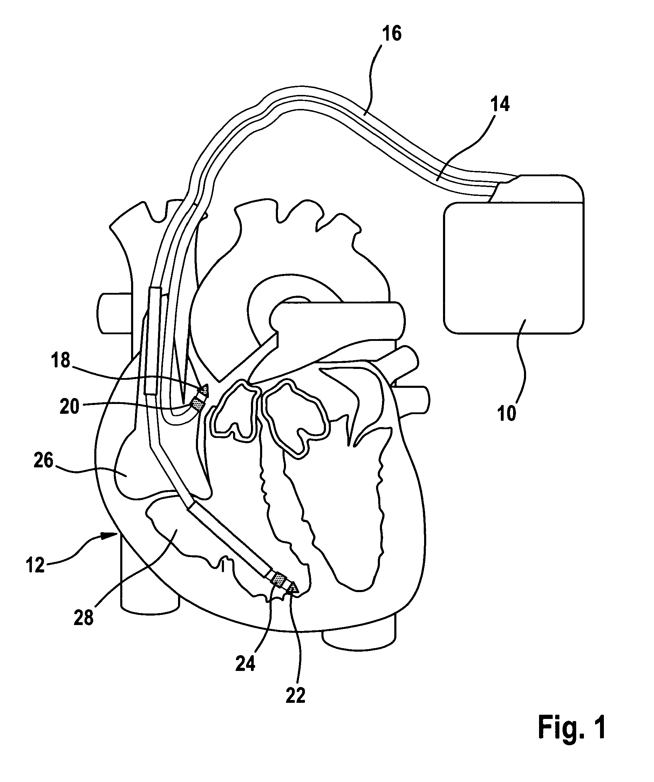

In FIG. 1 a dual chamber pacemaker 10 as implantable medical device connected to pacing/sensing leads placed in a heart 12 is illustrated. The pacemaker 10 is electrically coupled to heart 12 by way of leads 14 and 16. Lead 14 has a pair of right atrial electrodes 18 and 20 that are in contact with the right atria 26 of the heart 12. Lead 16 has a pair of electrodes 22 and 24 that are in contact with the right ventricle 28 of heart 12. Electrodes 18 and 22 are tip-electrodes at the very distal end of leads 14 and 16, respectively. Electrode 18 is a right atrial tip electrode RA-Tip and electrode 22 is a right ventricular tip electrode 22. Electrodes 20 and 24 are ring electrodes in close proximity but electrically isolated from the respective tip electrodes 18 and 22. Electrode 20 forms a right atrial ring electrode RA-Ring und electrode 24 forms a right ventricular ring electrode RV-Ring.

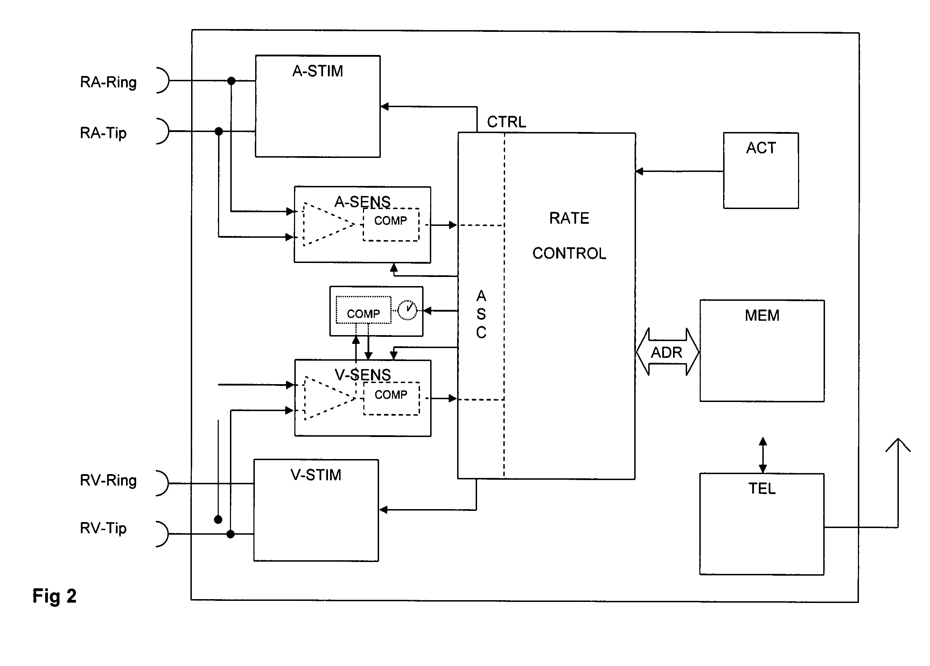

Referring to FIG. 2 a simplified block diagram of a dual chamber pacemaker 10 is illustrated. During operation of the pacemaker leads 14 and 16 are connected to respective output/input terminals of pacemaker 10 as indicated in Fig. 1 and carry stimulating pulses to the tip electrodes 18 and 22 from an atrial stimulation pulse generator A-STIM and a ventricular pulse generator V-STIM, respectively. Further, electrical signals from the atrium are carried from the electrode pair 18 and 20, through the lead 14, to the input terminal of an atrial channel sensing stage A-SENS; and electrical signals from the ventricles are carried from the electrode pair 22 and 24, through the lead 16, to the input terminal of a ventricular sensing stage V-SENS.

Controlling the dual chamber pacer 10 is a control unit CTRL that is connected to sensing stages A-SENS and V-SENS and to stimulation pulse generators A-STIM and V-STIM. Control unit CTRL receives the output signals from the atrial sensing stage A-SENS and from the ventricular sensing stage V-SENS. Sensing stage A-SENS generates an As detect signal indicating an atrial sense event as given by a P-wave each time the sensed atrial heart signal exceeds the detection threshold in a predetermined manner as further illustrated further below. Similarly, Sensing stage V-SENS generates a Vs detect signal indicating ventricular sense event as given by a R-wave each time the sensed ventricular heart signal exceeds the detection threshold of sensing stage V-SENS in a predetermined manner as further illustrated herein after. Thus, an As-signal is generated, when the atrial sensing stage A-SENS detects a P-wave and a Vs-signal is generated, when the ventricular sensing stage V-SENSE detects an R-wave.

Detection thresholds for ventricular and atrial sense events can be independently adjusted by control unit by adjusting the sensitivity of ventricular sensing stage V-SENS or atrial sensing stage A-SENS CTRL, respectively. Adjustment of the individual sensing stage's sensitivity can be performed by either adjusting the corresponding sense amplifier's gain or by adjusting the comparator's threshold value or both.

Control unit CTRL also generates trigger signals that are sent to the atrial stimulation pulse generator A-STIM and the ventricular stimulation pulse generator V-STIM, respectively. These trigger signals are generated each time that a stimulation pulse is to be generated by the respective pulse generator A-STIM or V-STIM. The atrial trigger signal is referred to simply as the "A-pulse", and the ventricular trigger signal is referred to as the "V-pulse". During the time that either an A-pulse or V-pulse is being delivered to the heart, the corresponding sensing stage, A-SENS and/or V-SENS, is typically disabled by way of a blanking signal presented to these sensing stage from the control unit CTRL, respectively. This blanking action prevents the sensing stages A-SENS and V-SENS from becoming saturated from the relatively large stimulation pulses that are present at their input terminals during this time. This blanking action also helps prevent residual electrical signals present in the muscle tissue as a result of the pacer stimulation from being interpreted as P-waves or R-waves.

Furthermore, atrial sense events As recorded shortly after delivery of a V-pulses during a preset time interval called post ventricular atrial refractory period (PVARP) are generally recorded but ignored.

Control unit CTRL comprises circuitry for timing ventricular and/or atrial stimulation pulses according to an adequate stimulation rate that can be adapted to a patient's hemodynamic need as pointed out below.

Still referring to FIG. 2, the pacer 10 may also include a memory circuit MEM that is coupled to the control unit CTRL over a suitable data/address bus ADR. This memory circuit MEM allows certain control parameters, used by the control unit CTRL in controlling the operation of the pacemaker 10, to be programmably stored and modified, as required, in order to customize the pacemaker's operation to suit the needs of a particular patient. Such data includes the basic timing intervals used during operation of the pacemaker. Further, data sensed during the operation of the pacer may be stored in the memory MEM for later retrieval and analysis.

A telemetry circuit TEL is further included in the pacemaker 10. This telemetry circuit TEL is connected to the control unit CTRL by way of a suitable command/data bus. Telemetry circuit TEL allows for wireless data exchange between the pacemaker 10 and some remote programming or analyzing device which can be part of a centralized service center serving multiple pacemakers.

The pacemaker 10 in FIG. 1 is referred to as a dual chamber pacemaker because it interfaces with both the right atrium 26 and the right ventricle 28 of the heart 10. Those portions of the pacemaker 10 that interface with the right atrium, e.g., the lead 14, the P-wave sensing stage A-SENS, the atrial stimulation pulse generator A-STIM and corresponding portions of the control unit CTRL, are commonly referred to as the atrial channel. Similarly, those portions of the pacemaker 10 that interface with the right ventricle 28, e.g., the lead 16, the R-wave sensing stage V-SENS, the ventricular stimulation pulse generator V-STIM, and corresponding portions of the control unit CTRL, are commonly referred to as the ventricular channel.

In order to allow rate adaptive pacing in a DDDR or a DDIR mode, the pacemaker 10 further includes a physiological sensor ACT that is connected to the control unit CTRL of the pacemaker 10. While this sensor ACT is illustrated in FIG. 2 as being included within the pacemaker 10, it is to be understood that the sensor may also be external to the pacemaker 10, yet still be implanted within or carried by the patient. A common type of sensor is an activity sensor, such as a piezoelectric crystal, mounted to the case of the pacemaker. Other types of physiologic sensors are also known, such as sensors that sense the oxygen content of blood, respiration rate, pH of blood, body motion, and the like. The type of sensor used is not critical to the present invention. Any sensor capable of sensing some physiological parameter relatable to the rate at which the heart should be beating can be used. Such sensors are commonly used with "rate-responsive" pacemakers in order to adjust the rate of the pacemaker in a manner that tracks the physiological needs of the patient.

In order to individually control a detection threshold of each of the sensing stages control unit CTRL is connected to the sense amplifiers V-SENS and A-SENS, respectively and adapted to control gain of the sensing amplifiers or the threshold value of corresponding comparator COMP or both. The higher the gain and the lower the comparator's threshold value, the lower is the detection threshold.

In order to avoid false detection of ventricular sense events by ventricular sensing stage V-SENS, the sensing stage is connected to a noise timer and a noise threshold comparator. The amplified ventricular heart signal is fed to the noise threshold comparator and is compared to a noise threshold value in order to determine whether or not the amplified ventricular heart signal exceeds the noise threshold value. The noise timer is connected to control unit CTRL and is started whenever the control unit CTRL triggers the ventricular stimulation pulse generator or when an ventricular sense event Vs is detected. The noise timer is further connected to the ventricular sensing stage and is adapted to prevent detection of any event by the ventricular sensing stage as long as the noise timer runs.

Control of the sensing amplifier's gain and/or the comparator's threshold value and thus the sensing stage's detection threshold is performed by the control unit CTRL as follows:
In the following description, magnitude of the heart signal is used, i.e. no consideration is given to the polarity of the picked-up signal.

A minimum threshold limit for the detection threshold is programmed - this is used as a boundary when adjusting the detection threshold automatically. This limit is also used as the final value when decreasing the dynamic detection threshold following the delivery of a pacing pulse. In the described embodiment, a default value of 0.25 mV is used as the minimum threshold limit.

A fixed amplitude threshold is used for categorizing complexes in two categories - those having high amplitudes and those having low amplitudes. In the described embodiment, the fixed amplitude threshold is 1.5 mV.

Subsequent to the detection of a heart complex using the dynamic threshold value in effect at that time, a detection hold-off period - having a programmable duration - is started and no further detections are made during this period. A default value of 121 ms is used for the detection hold-off period in the described embodiment.

During the detection hold-off period, the peak value of the complex is determined. Also, the complex is categorized as having high or low amplitude by comparing the peak value against the fixed amplitude threshold.

Following the expiration of the detection hold-off period, the dynamic threshold is set to the initial detection threshold value for a first detection period and, then, lowered gradually to finally reach the target detection threshold value for further detection periods. The initial detection threshold and the target detection threshold are programmable as percent of the peak of the detected heart complex. In the described embodiment, these percent values are 50% and 25% respectively. The dynamic threshold is never lowered to a value, which is below the programmed minimum threshold limit.

Various methods can be applied for lowering the dynamic threshold, e.g. lowering uniformly in steps or halving the value in each step or a combination of the two. In the described embodiment, the dynamic threshold is lowered by halving the value in a stepwise manner; the step duration and thus the duration of the detection periods is programmable having a default value of 125 ms.

If a heart complex is detected before the target detection threshold value or the minimum threshold value - if that is higher - has been reached, further lowering of the dynamic threshold is aborted.

The following part of the description further illustrates improvements the preferred embodiment of the invention provides over the prior art:

Similar to a sense event, also subsequent to a pace pulse delivery, a detection hold-off period - having a programmable duration - is started and no further detections are made during this hold-off period. However, no search for a peak amplitude value is performed during this hold-off period. A default value of 250 ms is used for this post-detection detection hold-off period in the described embodiment.

Following the expiry of the post stimulation pulse detection hold-off period, the initial detection threshold is set to a programmable value, and the target detection threshold is set to the minimum threshold value. The dynamic detection threshold is, then, set to the initial detection threshold value and, then, lowered gradually to finally reach the target detection threshold value. In the described embodiment, the post-pace initial detection threshold value is 1.5 mV.

After reaching the target detection threshold value and in case it is still higher than the minimum detection threshold value, an extra detection period having a longer duration is started, and subsequent to the expiry of that extra detection period, the dynamic detection threshold is set equal to the minimum detection threshold value. In the preferred embodiment, the duration of this extra detection period is 1 second.

The duration of the first detection period, used in the dynamic detection threshold lowering scheme, is multiplied by two if the most recent event is a paced event Vp or a detected heart complex (QRS complex comprising the R-wave) that has been identified as a low-amplitude complex because the peak amplitude of the detected R-wave does not exceed a preset peak amplitude threshold value.

A noise recognition mechanism is integrated with the described ASC feature. Following the detection of a heart complex (Vs) or following the delivery of a stimulation pulse (Vp), a noise timer with programmable duration (51 ms in this embodiment) is started. While this timer is running, no detections are possible. This timer is retriggered:
1. each time the polarity of the heart signal changes while the magnitude of the heart signal remains above the used noise threshold; or
2. each time the magnitude of the heart signal crosses the used noise threshold from below.

In figure 2, the first retriggering of the noise timer in all cases except the first case is due to change in the polarity and the only retriggering in the first case and the second retriggering in all other cases is due to threshold crossover from below (i.e. from the sub-noise-threshold zone).

The used noise threshold can be adaptive to the peak amplitude of the detected heart complex - e.g. 25% of the peak amplitude - or it can be a programmable or fixed value.
During any analog blanking that follows the delivery of a pace pulse or a shock, the started noise timer is kept frozen.

Following the detection of abnormal heart complexes, such as Premature Ventricular Contraction (PVC) in the ventricle and Far-field and Retrograde senses in the atrium, the target detection threshold value is not adapted to the peak values of such events - the target detection threshold value is left at its previous value. In the case of multiple PVCs or multiple Retrograde senses, this restriction applies only to the first one, as it could be start of a tachyarrhythmia.

The abnormal heart complexes as mentioned above are classified as such by other blocks of an implantable device and this classification is not part of the ASC feature.

If noise is suspected at the expiry of the detection hold-off period, the target detection threshold value is not adapted and the previous target detection threshold value is used - this is also the case even if it is a pace event, meaning that in this case, following the stimulation pulse, the final value of the dynamic detection threshold is not necessarily the minimum detection threshold rather it is what the target detection threshold was preceding the event. The noise is suspected if the noise timer was retriggered too close to the expiry of the detection holdoff period. In the preferred embodiment, "too close" is defined to be 12 ms.

The target detection threshold is not changed if the event is a Safety Window Pace pulse. The Safety Window Pace pulse is a commonly known bradycardia support feature.

In order to avoid declaring detection of a heart complex for a glitch or bit-noise in the signal, the disclosed ASC method has an option of requiring more than one consecutive samples being above the dynamic detection threshold before confirming detection. In the described embodiment, two consecutive samples - both above the dynamic detection threshold - are required to declare a detection.

## Claims

1. An implantable medical device for processing picked-up electric heart signals comprising:
a sensing stage connected or being connectable to an electrode for picking up electric potentials inside at least one chamber of a heart, said sensing stage having an adjustable sensitivity determining detection threshold, said sensing stage being further adapted to generate a detect signal indicating detection of a sense event if the time course of the picked-up signal exceeds the detection threshold,
a peak amplitude detector adapted to determine a peak amplitude of a picked-up heart signal,
a peak amplitude comparator that is connected to the peak amplitude detector and that is adapted to determine whether the peak amplitude detected by the peak amplitude detector exceeds a predetermined peak amplitude threshold value, and
a control unit that is connected to the peak amplitude comparator and to the sensing stage and that is adapted to adapt the detection threshold by adjusting the sensing stage's sensitivity,
wherein the control unit is adapted to
set the detection threshold for a first detection period to a high value corresponding to low sensitivity of the sensing stage following a detect signal generated by the sensing stage, and
subsequently lower the detection threshold stepwise for further detection periods, wherein either a first duration is used for the first detection period following a heart signal having an amplitude below the preset peak amplitude threshold or
a second duration is used for the first detection period following a heart signal having an amplitude above the preset peak amplitude threshold, said first duration being longer than said second duration.

2. The implantable medical device according to claim 1 further comprising:
a stimulation pulse generator adapted to generate electric stimulation pulses and being connected or being connectable to at least a stimulation electrode for delivering electric stimulation pulses to at least one chamber of the heart,
wherein the control unit further is connected to said stimulation pulse generator and is adapted to trigger the stimulation pulse generator to deliver stimulation pulses at scheduled points of time determined by the control unit.

3. The implantable medical device according to claim 1, wherein the high value of the detection threshold for the first detection period set after a detect signal is generated by the sensing stage is equal to a value that is a function of the peak amplitude of the picked up heart signal as determined by the peak amplitude detector.

4. The implantable medical device according to claim 2, wherein the control unit is adapted to apply the first duration for the first detection period following the delivery of a stimulation pulse.

5. The implantable medical device according to claim 2, wherein the control unit is further adapted to:
set a minimum detection threshold limit high enough to avoid detection of noise;
set a peak amplitude threshold for discrimination of large peak amplitude heart signals from low peak amplitude heart signals;
start a hold-off period each time the amplitude of a picked-up heart signal exceeds an actual detection threshold during which
(i) no detection can be made,
(ii) the peak amplitude of the heart signal is determined, and
(iii) the detected peak amplitude is categorized as to whether or not the peak amplitude exceeds said peak amplitude threshold value by comparing the detected peak amplitude against the said peak amplitude threshold value;
following the expiration of the hold-off period, to adjust the detection threshold to be equal to a value that is a function of the peak amplitude of the complex as searched and found according to the above;
subsequently to lower the detection threshold gradually to reach a lower final detection threshold value, the said lower detection threshold value being a function of the said detected peak amplitude value but never being lower than the said minimum detection threshold value;
start a hold-off period when a stimulation pulse is delivered during which no detection can be made;
adjust the detection threshold upon expiration of the holdoff period following a stimulation pulse to a fixed value high enough to avoid detecting evoked T-waves; subsequently to lower the detection threshold gradually to reach the final threshold value which is set equal to the said minimum detection threshold limit; and
abort the lowering of the detection threshold value if the threshold before reaching said minimum detection threshold value when the heart signal exceeds the detection threshold or when a stimulation pulse is delivered.

6. The implantable medical device according to claim 5, further comprising a noise timer and a noise threshold comparator for comparing the heart signal with a noise threshold value, said noise timer having the effect that no further detections are possible as long as the noise timer is running,
wherein the control unit is adapted to
start noise timer at the same time when the control unit starts said hold-off period, and
to restart said noise timer each time the polarity of the heart signal changes while the magnitude of it remains above a noise threshold, or the magnitude of the heart signal crosses the noise threshold from below.

7. The implantable medical device according to claim 6, wherein the said noise timer keeps its value frozen during any analog blanking that follows a delivery of a stimulation pulse or defibrillation shock.

8. The implantable medical device according to claim 1 or 5, wherein the sensing stage is adapted to sample the heart signal and to confirm detection of a sense event when more than one consecutive samples of the heart signal exceed the detection threshold.

9. The implantable medical device according to claim 1, wherein the implantable medical device is an implantable pacemaker or an implantable cardioverter/defibrillator.

10. An implantable medical device for processing picked-up electric heart signals comprising:
a sensing stage connected or being connectable to an electrode for picking up electric potentials inside at least one chamber of a heart, said sensing stage having an adjustable sensitivity determining detection threshold, said sensing stage being further adapted to generate a detect signal indicating detection of a sense event if the time course of the picked-up signal exceeds the detection threshold,
a peak amplitude detector adapted to determine a peak amplitude of a picked-up heart signal,
a peak amplitude comparator that is connected to the peak amplitude detector and that is adapted to determine whether the peak amplitude detected by the peak amplitude detector exceeds a predetermined peak amplitude threshold value, and
a control unit that is connected to the peak amplitude comparator and to the sensing stage and that is adapted to adapt the detection threshold by adjusting the sensing stage's sensitivity,
wherein the control unit is adapted to
set the detection threshold for a first detection period to a high value corresponding to low sensitivity of the sensing stage following a detect signal generated by the sensing stage, and
subsequently lower the detection threshold stepwise for further detection periods, to reach a lower final detection threshold value, the said lower final detection threshold value being a function of the said detected peak amplitude value but never being lower than the said minimum detection threshold value;
wherein the control unit is further adapted to decide either to adapt or not to adapt said final detection threshold value based on the type or classification of the sensed or paced heart event
and to maintain a previously found final detection threshold value in case the control unit decides not to adapt said final detection threshold value.

11. The implantable medical device according to claim 10, wherein the control unit is adapted to decide not to adapt or adjust said final detection threshold value if one or more of the following event types are detected and instead a previous value of the final detection threshold value is used as a target detection threshold value when lowering the detection threshold:
- a single premature ventricular contraction or first premature ventricular contraction in a sequence of premature ventricular contractions,
- a single detection in the retrograde window or first detection in a sequence of such detections
- a Far-field detection of a ventricular complex in the atrium
- a delivery of a safety window pace pulse
- a delivery of a pace pulse while the said noise timer is running
- if noise is suspected at the expiry of the hold-off period following detection of a sense event.

12. A method of controlling sensitivity of a sensing stage of an implantable medical device comprising a sensing stage connected or being connectable to an electrode for picking up electric potentials inside at least one chamber of a heart, said sensing stage having an adjustable sensitivity determining detection threshold, said sensing stage being further adapted to generate a detect signal indicating a sense event if the time course of the picked-up signal exceeds the detection threshold,
a peak amplitude detector adapted to determine a peak amplitude of a picked-up heart signal,
a peak amplitude comparator that is connected to the peak amplitude detector and that is adapted to determine whether the peak amplitude detected by the peak amplitude detector exceeds a predetermined peak amplitude threshold value, and
a control unit that is connected to the peak amplitude comparator and to the sensing stage and that is adapted to adapt the detection threshold by adjusting the sensing stage's sensitivity
said method comprising the steps of:
Setting a minimum detection threshold limit high enough to avoid detection of noise;
Setting a peak amplitude threshold for discrimination of large peak amplitude heart signals from low peak amplitude heart signals;
Starting a holdoff period each time the amplitude of a picked-up heart signal exceeds an actual detection threshold during which
(i) no detection can be made,
(ii) the peak amplitude of the heart signal is determined, and
(iii) the detected peak amplitude is categorized as to whether or not the peak amplitude exceeds said peak amplitude threshold value by comparing the detected peak amplitude against the said peak amplitude threshold value;
following the expiration of the hold-off period, Adjusting the detection threshold to be equal to a value that is a function of the peak amplitude of the complex as searched and found according to the above;
subsequently Lowering the detection threshold gradually to reach a lower final detection threshold value, the said lower final detection threshold value being a function of the said detected peak amplitude value but never being lower than the said minimum detection threshold value;
Starting a hold-off period when a stimulation pulse is delivered during which no detection can be made;
Adjusting the detection threshold upon expiration of the holdoff period following a stimulation pulse to a fixed value high enough to avoid detecting most evoked T-waves;
subsequently lowering the detection threshold gradually to reach the final detection threshold value which is set equal to the said minimum detection threshold limit; and
Aborting the lowering of the detection threshold value if the threshold before reaching said minimum detection threshold value when the heart signal exceeds the detection threshold or when a stimulation pulse is delivered.

13. The method according to claim 12 where gradual decrease in the detection threshold is achieved by decreasing the detection threshold value to a fixed percent of the preceding detection threshold value in each step, by making uniform decreases in each step or by a combination of both.

14. Method as in claim 12 or 13 comprising the steps of
comparing a detected peak amplitude of a picked-up heart signal with said peak amplitude threshold value and
applying either a first duration for the first detection period if the peak amplitude of the heart signal does not exceed the peak amplitude threshold value, or
applying a second duration for the first detection period if the peak amplitude of the heart signal does exceed the peak amplitude threshold value,
said first duration being longer than said second duration.

15. The method according to claim 14 wherein a longer duration than said second duration is used for the first detection period after delivery of a stimulation pulse.

16. The method according to claim 14 where, for the case of a detection and if the final threshold value reached is still higher than the minimum threshold value, the threshold is lowered to the minimum threshold value following an extra detection period having a longer duration than said second duration.

17. The method according to claim 12 wherein a retriggerable noise timer is started at the same time when the said hold-off period is started and where no further detections are possible as long as this timer is running and wherein said noise timer is retriggered each time the polarity of the signal changes while the magnitude of it remains above a noise threshold, or the magnitude of the signal crosses the noise threshold from below.

18. The method according to claim 17 where the said noise threshold is either a fixed value, or a function of the detected peak value and equals the final detection threshold value.

19. The method according to claim 17 where the said noise timer keeps its value frozen during any analog blanking that follows a delivery of a stimulation pulse or defibrillation shock.

20. The method according to claim 12 wherein the heart signal is sampled and
wherein detection of a heart complex is confirmed when more than one consecutive samples of the heart signal exceeds the detection threshold.

21. A method of controlling sensitivity of a sensing stage of an implantable medical device comprising a sensing stage connected or being connectable to an electrode for picking up electric potentials inside at least one chamber of a heart, said sensing stage having an adjustable sensitivity determining detection threshold, said sensing stage being further adapted to generate a detect signal indicating a sense event if the time course of the picked-up signal exceeds the detection threshold,
a peak amplitude detector adapted to determine a peak amplitude of a picked-up heart signal,
a peak amplitude comparator that is connected to the peak amplitude detector and that is adapted to determine whether the peak amplitude detected by the peak amplitude detector exceeds a predetermined peak amplitude threshold value, and
a control unit that is connected to the peak amplitude comparator and to the sensing stage and that is adapted to adapt the detection threshold by adjusting the sensing stage's sensitivity
said method comprising the steps of:
Setting a minimum detection threshold limit high enough to avoid detection of noise;
Setting a peak amplitude threshold for discrimination of large peak amplitude heart signals from low peak amplitude heart signals; and
subsequently Lowering the detection threshold gradually to reach a lower final detection threshold value, the said lower final detection threshold value being a function of the said detected peak amplitude value but never being lower than the said minimum detection threshold value;
wherein the said final detection threshold value is not adapted or adjusted if one or more of the following event types are detected and instead a previous value of the final detection threshold value is used as a target detection threshold value when lowering the detection threshold:
- a single premature ventricular contraction or first premature ventricular contraction in a sequence of premature ventricular contractions,
- a single detection in the retrograde window or first detection in a sequence of such detections
- a Far-field detection of a ventricular complex in the atrium
- a delivery of a safety window pace pulse
- a delivery of a pace pulse while the said noise timer is running
- if noise is suspected at the expiry of the hold-off period following detection of a sense event.

22. The method according to claim 21 wherein noise is suspected at the expiry of the hold-off period if the noise timer was retriggered too close to the expiry of the hold-off period.
